Europäisches Patentamt

European Patent Office

Office européen des brevets

(11) Publication number: **0 375 075**
**A1**

## EUROPEAN PATENT APPLICATION

(21) Application number: **89203278.0**

(22) Date of filing: **21.12.89**

(51) Int. Cl.⁵: **C12P 33/00, C12N 15/52, C12N 1/21, C12N 15/78, C12P 1/00, //(C12N1/21, C12R1:38)**

(30) Priority: **22.12.88 NL 8803141**

(43) Date of publication of application:
**27.06.90 Bulletin 90/26**

(84) Designated Contracting States:
**AT BE CH DE ES FR GB GR IT LI LU NL SE**

(71) Applicant: **NEDERLANDSE ORGANISATIE VOOR TOEGEPAST-NATUURWETENSCHAPPELIJK ONDERZOEK TNO**
**Juliana van Stolberglaan 148**
**NL-2595 CL The Hague(NL)**

(72) Inventor: **Hesselink, Paulus Gerhardus Maria**
**Irisstraat 31**
**NL-6666 BE Heteren(NL)**
Inventor: **te Velthuis, Jan Hendrik**
**Prinsenstraat 45**
**NL-9711 CL Groningen(NL)**
Inventor: **Witholt, Bernard**
**Anemoonweg 44**
**NL-9765 HE Paterswolde(NL)**

(74) Representative: **Smulders, Theodorus A.H.J., Ir. et al**
**Vereenigde Octrooibureaux Nieuwe Parklaan 107**
**NL-2587 BP 's-Gravenhage(NL)**

(54) **A process for the microbiological preparation of steroids; genetically modified microorganisms suitable therefor.**

(57) The invention relates to a process for preparing steroids, in particular C-17 ketosteroids, by means of steroid side chain oxidation using genetically modified microorganisms, and to genetically modified microorganisms which are suitable therefor. According to the invention, said microorganisms are the result of a transfer of genetic material between two bacterial species, preferably from Actinomycetales to Pseudomonads.

FIG.1

**A process for the microbiological preparation of steroids; genetically modified microorganisms suitable therefor.**

This invention relates to a process for preparing steroids, particularly C-17 ketosteroids, by means of steroid side chain oxidation through genetically engineered microorganisms.

Steroid hormones, such as sex hormones, and corticosteroids are increasingly synthesized from naturally occurring steroids containing a C-17 side chain. The substrates employed in this connection are particularly the sterols cholesterol, $\beta$-sitosterol and campesterol. By completely or partly removing the C-17 side chain there are formed C-17 ketosteroids, such as androst-4-en-3,17-dione (AD) and androsta-1,4-dien-3,17-dione (ADD) or C-21/22 pregnane derivatives, such as pregnenolonoids and bisnorcholest-4-en-3-on-22-carboxylic acid (BNC). These products are very suitable for a further conversion to substantially all the desired steroid final products, as described by G.R. Lenz (Steroids, in: Kirk-Othmer Encyclopaedia of Chemical Technology, 3rd edition, Wiley Interscience, New York, 21, 645-729 (1983)).

The removal or oxidation of the steroid C-17 side chain must be carried out with microorganisms, since this is not possible with purely chemical reactions in an economically profitable manner. The microbial oxidation of the side chain occurs stepwise. If cholesterol is the substrate, cholestenone is first formed by the enzyme cholesterol oxidase. After a first series of oxidative reactions at the terminal carbon atom of the side chain, cholestenone C-27 carboxylic acid is formed. Subsequently, propionic acid is separated to form 3-ketochol-4-enic acid. By separating acetate BNC is formed therefrom, which is already a valuable product. Through a last series of oxidative reactions propionic acid is separated once more to form AD. The reactions of this last mentioned series may also occur under anaerobic conditions, in contrast to the earlier mentioned reactions. By the action of a dehydrogenase ADD is formed from AD. The process steps and the enzymes involved are described in reviews, inter alia by C.K.A. Martin (Sterols, in: Biotechnology 6A, K. Kieslich (editor), Verlag Chemie, Weinheim, West Germany, pages 79-95 (1984)). If $\beta$-sitosterol and/or campesterol function as substrates, the carbon atoms C-28-29 or C-28 are first removed from the cholesterol side chain before the above mentioned reaction scheme is followed. The multienzyme complex responsible for this is described by C.S. Chen in his thesis (The mechanism of degradation of side chains of phytosterols by microorganisms, University of Wisconsin, Madison, USA (1985)).

A drawback of the microbial side chain breakdown is the occurrence of secondary reactions, such as the steroid nucleus degradation. In the degradation of the nucleus the steroid skeleton composed of four rings is broken down irreversibly, and there are formed compounds which have lost their activity as steroid hormones. The nucleus degradation route is described in the review by K. Kieslich (Microbial side chain degradation of sterols, Journal of Basic Microbiology, 25, 461-474 (1985)). At present such undesirable secondary reactions can only be suppressed efficiently by using inhibitors of the enzymes involved in the nucleus degradation. This increases the cost of the procedure, while an absolute blockage of the secondary reactions causing product loss is usually not obtained.

A further drawback results from the fact that nearly all the hitherto known microorganisms capable of steroid side chain break down belong to the bacterial order Actinomycetales. Examples thereof are Arthrobacter, Brevibacterium, Corynebacterium, Mycobacterium, Nocardia, Rhodococcus and Streptomyces. The microorganisms suitable for steroid side chain breakdown are isolated chiefly from nature, and some times they are treated as an intact organism with a mutagenic compound or with ultraviolet light in order to eliminate or reduce an undesirable property. These genetic engineering techniques are very non-directed, since they damage the complete DNA and are mostly associated with the generation of secondary mutations. Examples of microorganisms constructed by these techniques and described in the literature are Mycobacterium sp. NRRL-B 3683 and NRRL-B 3805 and the Corynebacterium sp. ATCC 31458, ATCC 31459 and ATCC 31460 (Kraychy, Marsheck, and Muir, Selective microbiological degradation of steroidal 17-alkyls, US-A-3,684,657 (1970); and Hill, Schindler, Schmid, Wagner, and Voetter, Microbial conversion of sterols; selection of mutuants for production of 20-carboxy-pregna-1,4-dien-3-one, European Journal of Applied Microbial Biotechnology, 15, 25-32 (1982)). In general, the representatives of the above bacterial order grow slowly; the rates of growth are usually below 0.1 (hour)$^{-1}$, corresponding to generation times of more than 7 hours. For comparison: most of the Pseudomonas species grow at a rate of about 0.45 (hour)$^{-1}$ or a generation time of 1.5 hours. Furthermore, many species tend to coagulate, float and adhere to reactor walls, measuring instruments and supply and discharge conduits in the fermenter. This results in long waiting times for obtaining sufficient biomass and in seriously hampered process conditions. Besides, these bacteria often grow only in media containing relatively expensive components, such as yeast extract and/or protein lysates, as a source of essential growth factors. This leads to an expensive procedure.

Another drawback of the microbial steroid side chain breakdown resides in the fact that steroids are poorly water-soluble. The solubility of cholesterol in an aqueous medium is 1.8 mg/l, that of AD and ADD about 30-50 mg/l. For the steroid side chain breakdown to be carried out in an economically profitable manner, the substrate concentration must be a few grams per liter. At such concentrations there are formed substrate crystals, which results in a substrate inaccessible to the microorganism. Moreover, there are formed substrate-product mixed crystals. This results in a suboptimum procedure. Also, the rate at which sterols go into solution is very low in aqueous media, so that dissolved substrate reacted away is only slowly supplemented by dissolving substrate. Attempts have been made to increase the solubility and rate of dissolving of the substrates in aqueous media by creating microcrystals by means of grinding, ultrasonication or by adding the substrate to the fermenter in a heated solvent and by adding detergents and emulsifying compounds, such as Tween, Brij and Span. This proved to be a solution to a limited extent only, since the above compounds have a toxic side effect on the microorganisms at those concentrations at which the solubility and rate of dissolving of the substrates is strongly increased. Besides, attempts have been made to carry out the steroid side chain breakdown in organic solvents in which the substrates and products are properly soluble, by means of cells immobilized in hydrophilic gelling compounds, such as alginate, chitosan and carrageenan. These attempts were also proved unsuccessful, since both the substrates and the oxygen required could only very slowly penetrate into the immobilized cells because of diffusion limitation. Furthermore, in the immobilization matrix the cells were under conditions inhibiting the cell growth. Some microorganisms, however, are known to substantially break down the steroid side chain during their growth phase. Finally, it also turned out that the toxic side effect of the solvents employed through immobilization was not quite avoidable. This together led to slow transformations and economically unprofitable processes.

This invention provides a process for the accelerated realization of steroid transformations, particularly the steroid C-17 side chain breakdown, in an aqueous medium or in a medium comprising one or more organic solvents or in a multi-liquid phase system, by means of genetically modified microorganisms, such as Pseudomonas species, and a process for the construction of microorganisms capable of realizing the above steroid transformations in the reaction conditions in question. The construction of the desired mutants occurs in principle by transferring selected fragments of DNA from a microorganism capable of desired steroid transformations but having the above drawbacks to host microorganisms which previously were not capable of these steroid conversions but, for the rest, would be appropriate for rapid steroid conversions, such as Pseudomonas species. This is the fundamental difference between this process and the non-directed mutagenesis of intact microorganisms without transfer of DNA between bacteria, as described by Kraychy et al. and Hill et al..

Although, in principle, many kinds of genetic engineering techniques are known, a method of transferring genetic material from a Mycobacterium to Pseudomonas species, allowing this material to stably inherit and expressing it, has not been known so far. Furthermore, a method of tracing, identifying and subcloning the genes responsible for steroid transformations has not been known so far either. Surprisingly, it has been found that a cloning system in which parts of Mycobacterium sp. NRRL-B 3683 DNA were incorporated into the cosmid pLAFRI (Friedman et al., Gene 18, 289-296 (1982)), followed by in vitro packaging of this DNA construct by bacteriophage lambda, followed by introduction of the genetic material into E. coli, followed by conjugation of these E. coli's with Pseudomonas testosteroni, P. oleovorans or P. putida via bi- or triparental mating experiments, led to the introduction of this material into the above Pseudomonas species with a high frequency. Furthermore, it has surprisingly been found that selection of the desired steroid transforming genes could occur by subjecting P. testosteroni recombinants which were provided with parts of the Micobacterium DNA to growth tests on those steroids as the only source of carbon on which a P.testosteroni could not grow without this additional genetic material. Finally, it has surprisingly been found that the resulting recombinant Pseudomonas species were capable of conversions of steroids as well as of other branched and unbranched hydrocarbons which were previously unknown for these microorganisms. These conversions could occur both in aqueous fermentation systems and in systems containing one or more organic solvents, if required added as an additional phase. The invention will be explained by reference to some examples.

Examples

1. Genetic engineering.

a. A survey of the employed strains is to be found in Table I. This table gives the names of the employed bacteria, some relevant properties, the number under which they are deposited with official strain collections and a reference to scientific literature concerning these strains.

TABLE I

| strain | relevant genotype | source or reference |
|---|---|---|
| Mycobacterium sp. | steroid transformation | NRRL-B 3683 |
| | sterol side chain cleavage | ATCC 29472(1) |
| P.testosteroni | growth on C19-C21 steroids | ATCC 11996(5) |
| P.oleovorans GPo-1 | alkane oxidation, resistance to organic solvents, alk BAC/R on the OCT plasmid | ATCC 29347(2) |
| P.oleovorans GPo-12 | as GPo-1 without OCT plasmid | (2) |
| P.putida P$_p$KT-mtz | | (3) |
| P.putida P$_p$G -1 | CAMOCT plasmid | (2) |
| E.coli strains: | | |
| HB 101 | recA$^-$, hsdR$^-$, hsdM$^-$, pro, leu, str$^-$ | (6) |
| GEc 47 | as HB 101, pGEc 47 (pLAFRI:: (alkBAC::alkR) | (7) |
| XJS 5037 | as HB 101, pRK 2013 (Km$^r$,Tra$^+$,Mob$^+$) | (8) |
| SF 800 | W 3110, Nal$^r$, polA$^-$, thy | (9) |
| BHB 2688 | red 3 (in phage λ2) D$^-$ | (6) |
| BHB 2690 | red 3 (in phage λ2) E$^-$ | (6) |

Abbreviations: ATCC = American Type Culture Collection, Rockville MD, USA; NRRL = Northern Utilization Research and Development Division, Peoria Il., USA.

References:

1. Marsheck, Kraychy, and Muir (1972), Microbial degradation of sterols, Appl. Microbiol. 23, 72-77.

5. Talaly, Dobson, and Tapley (1944), Oxidative degradation of testosterone by adaptive enzymes, Nature 170, 620-621.

6. Maniatis, Fritsch, and Sambrook (1982), Molecular cloning, a laboratory manual, Cold Spring Harbor Laboratory, Cold Spring Harbor, New York.

2. Eggink (1987): Genetics of alkane degradation in Pseudomonas oleovorans PhD thesis University of Groningen, Groningen, The Netherlands.

3. Timmis et al (1981) Gene 16, 237.

7. Eggink, Lageveen, Altenburg, and Witholt (1987), Controlled and functional expression of the Pseudomonas oleovorans alkane utilizing system in Pseudomonas putida and Escherichia coli, J. Biol. Chem. 262, 17712-17718.

8. Figurski, and Helinsky (1979), Replication of an origin containing derivate of plasmid RK2 dependent on a plasmid function provided in trans. Proc. Natl. Acad. Sci. USA 76, 1648-1652.

9. De Lucia, and Cairns (1969), Isolation of an E.coli strain with a mutation affecting DNA polymerase, Nature 224, 1164-1168.

b. The bacteria were cultured on the following media and under the following conditions. E. coli strains were cultured on L-medium at 37°C and a pH of 7, and at 200 rpm on a standard shaker. Pseudomonas species and Mycobacterium were cultured on L- or E2-medium, the latter optionally provided with a suitable carbon source, such as glucose, citrate or succinate, in a concentration of from 1 to 5 grams per litre, at a temperature of 30-32°C and the above pH and shaking conditions. In the case of solid nutrient media 15 grams of agar per litre were added to the above media prior to sterilization for 30 minutes at 121° C. Antibiotics were used in the following concentrations: tetracycline 15 mg/l; kanamycin 25 mg/l; streptomycin 20 mg/l; nalidixic acid 20 mg/l.

L-medium contains:

5 g/l yeast extract

10 g/l bacto-tryptone

5 g/l NaCl

the pH is adjusted to 7.2 .

E2-medium contains:

3.5 g/l $NaNH_4HPO_4.4 H_2O$

7.5 g/l $K_2HPO_4.3 H_2O$

3.7 g/l $KH_2PO_4$

After sterilization there is added to 1 l $E_2$-medium 10 ml of a sterile 100 mM $MgSO_4$ solution and 1 ml of a trace element solution called 1000 x MT. This 1000 x MT contains per litre:

2.78 g $FeSO_4 . 7H_2O$

1.98 g $MnCl_2 . 4H_2O$

2.81 g $CoSO_4 . 7H_2O$

1.47 g $CaCl_2 . 2H_2O$

0.17 g $CuCl_2 . 2H_2O$

0.29 g $ZnSO_4 . 7H_2O$

in 1 N HCl.

c. DNA isolations and analyses were conducted as follows. The total chromosomal and, if required, plasmid DNA of Mycobacterium sp. NRRL-B 3683 was isolated according to a modified method of Clark-Curtiss et al. (Molecular analysis of DNA and construction of genomic libraries of Mycobacterium leprae, Journal of Bacteriology 161, 1093-1102 (1985)). These modifications served to obtain largest possible intact DNA fragments. In practice, this meant that only siliconized glassware was used during isolation; that after alcohol precipitation from solutions DNA was removed by means of adhesion to Pasteur pipettes instead of by centrifugation; that DNA was further purified by carrying out two gradient centrifugations in the presence of cesium chloride immediately after each other; that DNA was removed from the resulting cesium chloride gradient centrifuge tubes by means of pipettes having a wide (at least 3 mm in cross-section) opening instead of injection needles as usual. The DNA finally obtained by this method had lengths of 50-100 kb on an average and was free of protein or single-stranded ruptures. Plasmid DNA was isolated according to the method by Birnboim and Doly (Nucleic Acid Research 7, 1513-1523 (1979) and was further purified, if required, by means of cesium chloride gradient centrifugation according to Maniatis et al..

The restriction endonucleases EcoRI and BamHI were obtained from Boehringer (Mannheim, FRG) and SalI was obtained from Bethesda Research Laboratories (Neu Isenheim, FRG).
These enzymes were used as prescribed by their suppliers. The analysis of DNA fragments was carried out by means of agarose gel electrophoresis as described by Maniatis et al.

d. The construction of a gene bank consisting of parts of Mycobacterium DNA in the cosmid pLAFRI and the transfer of this DNA to E. coli occurred as follows. The Mycobacterium DNA purified by the above described procedure was subjected to partial digestion with EcoRI. The plasmid pLAFRI purified by means of a cesium chloride gradient was cut completely with EcoRI. Both types of DNA fragments were brought together in a ratio (of vector DNA : insertion DNA) of 2 : 1 on a weight basis and in a final DNA concentration of 750 µg/ml, after which T4 DNA ligase was added as described by Maniatis et al.. The ligation occurred for 2 hours at room temperature. At the termination of this reaction an in vitro packaging reaction was conducted with 1 µl of the resulting DNA solution, as described by Maniatis et al., and the E. coli strains HB 101 or XJS 5037 were infected by the resulting recombinant baateriophages lambda. Thus, about 1800 tetracycline resistant E. coli transductants were obtained per µl of the employed DNA solution. In total, 32,000 transductants were thus obtained, of which may be deemed sufficient for tracing a gene with a chance of success above 99 %.

When checking the resulting E. coli recombinants, it turned out that an average of 18 % of these strains contained a fragment of Mycobacterium DNA of about 23 kb, 45 % fragments of about 7.5 kb and 36 % only pLAFRI dimers without Mycobacterium DNA.

e. The Mycobacterium DNA was inserted into P. testosteroni and P. oleovorans as follows. The same procedure was followed in the transfer of Mycobacterium DNA to other bacteria, such as P. putida.

The plasmid pLAFRI, with or without DNA insert, can in principle be transferred to another bacterium by inserting the plasmid pRK 2013 into the pLAFRI containing bacterium, followed by contacting the strain with these two plasmids with a suitable recipient strain. This conjugation procedure is described by Friedman et al. (Gene 16, 289-296 (1982)). Two new routes were developed to introduce DNA originating from Mycobacterium into Pseudomonas species. In the first case the donor strain employed was E. coli HB 101 infected by bacteriophage lambda. The recombinant E. coli's HB 101 obtained under 1d. were spread on an agar plate with L-medium and grown overnight at 37°C. Subsequently, this plate was replicated on a plate with L-medium containing a thin layer of E. coli XJS 5037, and this plate was incubated overnight at 37°C. Then the last mentioned plate was replicated on a plate with L-medium and with a thin layer of P. testosteroni. The resulting plate now containing 3 bacterial species was incubated for 8 hours at room temperature. Subsequently, this plate was replicated on a plate with E2-medium, 2 g/l citrate as a carbon source and 15 mg/l tetracycline. All the E. coli species employed cannot grow on this medium, nor can P. testosteroni strains without pLAFRI. An efficiency of plasmid transfer from E. coli to P. testosteroni of about 1 to 10,000 could thus be realized.

Since about 1 million E. coli's were employed per DNA transfer experiment, this was amply sufficient for introducing DNA into P. testosteroni.

In the second case E. coli XJS 5037 was directly infected by bacteriophage lambda, and introduction of Mycrobacterium DNA into P. testosteroni could occur via a so-called biparental mating. In this case the frequency of pLAFRI transfer was about one factor of 10 higher than in the procedure first described.

2. Selection of mutants containing DNA fragments coding for steroid transformations, such as the steroid C-17 side chain oxidation.

A genetically unmodified P. testosteroni is capable of using steroids having a short C-17 side chain as the only carbon source. These comprise AD, ADD, testosterone, progesterone and pregnenolone. BNC is on the verge of what is still metabolizable for P. testosteroni; 5-cholenic acid, cholest-4-en-3-one and cholesterol cannot be utilized. In order to test whether steroid transforming genes of Mycobacterium had been transferred to the P. testosteroni recombinants obtained under 1. and whether they could express in this host, P. testosteroni mutants were plated on agar plates with E2-medium to which 200 mg/l of a steroid as the only carbon source were added and incubated for one week at 30°C. This selection step was repeated several times, and finally 22 mutants were thus recognized out of 2000 tested strains having modified growth properties. A survey of the growth properties of a plurality of these strains is given in Table II.

3. Steroid fermentations by P. testosteroni recombinants in aqueous medium.

Of a plurality of P. testosteroni recombinants it was examined whether, in addition to growth on steroids, they were also capable of C-17 side chain breakdown of cholesterol. 250 ml Erlenmeyer flasks with 50 ml L-medium and 1 g/l cholesterol were suitably inoculated with P. testosteroni recombinants. Samples were regularly taken and examined for cell growth and steroid content, respectively by means of optical density measurement at 450 nm and capillary gas chromatography / gas chromatography mass spectrometry. Some strains produced an unidentified steroid and caused a strong nucleus degradation of cholesterol. Also, traces of AD and ADD were found. The genetically unmodified P. testosteroni strain did not prove capable of this, so that it may be concluded that the transferred Mycobacterium genes were responsible for these effects (Table II). That only low concentrations of AD and ADD were found can easily be accounted for by the fact that each P. testosteroni strain, both genetically modified and unmodified, can excellently utilize these two steroids as carbon source. This degradation of the desired products can in principle be inhibited by adding inhibitors for the nucleus degradation, as described in the introductory part. By way of alternative for suppressing the nucleus degradation, so-called minus-mutants of Pseudomonas species that are no longer capable of nucleus degradation can be made by means of genetic techniques.

## TABLE II

Growth on different steroids and cholesterol transformation by P. testosteroni recombinants. Good growth or accelerated growth (BNC) is indicated by +, weak growth by ± and absence of growth by -. The formation of the unidentified steroids after cholesterol fermentation for 24 hours is indicated in % disappearance of the initial molar amount of cholesterol (2.59 mM). Abbreviations used: chol = cholesterol, $\Delta^4$-cholon = $\Delta^4$-cholestenone, $\Delta^5$-ca = $\Delta^5$-cholenic acid, BNC = bisnorcholest-4-en-3-one-22-carboxylic acid, nucl.deg. = nucleus degradation, wt = wild type, nd = not determined.

| | chol | growth characteristics | | | cholesterol fermentation | |
| | | $\Delta^4$-cholon | $\Delta^5$-ca | BNC | product(%) | nucl.deg.(%) |
| --- | --- | --- | --- | --- | --- | --- |
| s t r a i n | | | | | | |
| wt | - | - | - | - | 0 | 0 |
| HVH A3 | + | + | + | - | 0 | < 10 |
| HVH A4 | ± | ± | ± | - | 0 | nd |
| HVH A9 | + | + | + | - | 0 | nd |
| HVH A10 | + | + | + | - | 7.2 | 35 |
| HVH A27 | - | + | + | - | 5.3 | 0 |
| HVH A34 | + | + | + | + | 0 | nd |
| HVH A36 | - | - | + | - | 5.8 | 20 |
| HVH A44 | + | ± | + | - | 7.7 | 50 |
| HVH A50 | ± | ± | ± | ± | 7.2 | 0 |
| HVH B11 | ± | + | + | + | 0.8 | nd |
| HVH B13 | - | + | ± | ± | 5.3 | nd |
| HVH B14 | - | - | + | - | 1.5 | nd |

7

4. Characterization of DNA fragments involved in steroid transformations.

Of a number of P. testosteroni mutants the plasmid introduced into these strains under 1. was examined. Because isolation of plasmid DNA from Pseudomonas proceeds difficultly, the plasmids were isolated from the E. coli strains which had served as plasmid donor. The DNA was examined by means of agarose gel electrophoresis after treatment with restriction enzymes. It was found that most of the plasmids responsible for changed growth and steroid transformation properties in P. testosteroni recombinants contained a part of a common piece of Mycobacterium DNA of about 12.45 kb in length. Fig. I shows this piece of DNA and indicates the parts that were made responsible for changed growth properties for nucleus degradation and product formation.

5. Other new growth characteristics of the resulting recombinant Pseudomonads.

From a chemical point of view, the sterol side chain is to be understood as a branched alkane. The microbial oxidation of the two types of compounds proceeds analogously. Consequently, there are hypotheses that possibly a number of the same enzymes are involved in these oxidations. Therefore, it was examined whether the introduction of Mycobacterium genes into P. testosteroni had led to an extension of the number of alkanes and related hydrocarbons that could serve as the only source of carbon.

It was found that P. testosteroni HVHA 44 was indeed capable of growing on pentane, hexane, heptane, octane, nonane, decane, dodecane, tetradecane, hexadecane, pristane* and 2-propanol, in contrast to a genetically unmodified P. testosteroni. All these compounds are suitable as the only growth substrate for Mycobacterium sp. NRRL-B 3683. It was further found that the above mutant HVHA 44 was capable of using 2.3-dimethylbutane*, 2.5-dimethylpentane*, cyclohexane and 1-octene as a growth substrate, compounds that could not be used as the only source of carbon by Mycobacterium or by the wild type P. testosteroni either. Especially the ability of breaking down the branched alkanes marked with * is interesting since these strongly resemble sterol C-17 side chains. Apparently, the right combination of Mycobacterium and P. testosteroni genes leads to the expression of a said set of enzymes capable of oxidizing branched hydrocarbons, including sterol side chains.

6. Growth of P. testosteroni mutants in an organic solvents medium.

P. testosteroni recombinants offer the possibility referred to under 3. of accelarated steroid conversion in aqueous medium because of the much higher growth rates of these strains when compared with Mycobacterium sp. NRRL-B 3683 (doubling times of respectively 1.5 - 2 hours versus 7 hours in L-medium). Besides, it is interesting to further increase steroid conversion rates by increasing the steroid solubility, e g., by using an organic solvent as a second phase in the fermenter. The background idea is that steroid molecules pass from this organic phase into the aqueous phase, reach the bacterium and are converted into a product, and that this product diffuses back again into the organic phase. Thus, the maximum dissolved substrate concentration in the aqueous phase can be maintained continuously. Also, the inhibiting effect, if any, exerted by a product on its own formation, is thereby avoided since also the product will dissolve in the organic solvent to a better degree and will therefore not be present in the aqueous phase in an unduly high concentration. However, many microorganisms are not resistant to organic solvents and show no growth or product formation in such a medium.

According to the invention, however, a further examination has shown that P. testosteroni HVHA 44 was capable of properly growing in a two-phase system in which 10 - 50% pristane or hexadecane on a volume base was added to the aqueous phase. This opens the possibility of carrying out steroid C-17 side chain breakdown with the resulting mutants or with other bacterial strains into which the right genes are introduced in a multi-phase system containing one or more solvents in which steroids better dissolve than in water.

7. Description of process conditions for carrying out steroid transformations by means of recombinant Pseudomonads in an organic solvents medium.

In the thesis by R.G. Lageveen (Oxidation of aliphatic compounds by Pseudomonas oleovorans, University of Groningen (1986)) and in Dutch Patent Application 8700085 (A process for preparing compounds with a terminal hydroxyl or epoxy group; microorganisms suitable therefor) the conditions are described in which P. oleovorans and some P. putida strains properly grow and form a product in multi-phase sytems. These conditions comprise: pH = 6 - 8, temperature = 25 - 37° C, 1 - 99.5 % organic phase consisting of one or more organic solvents on a volume basis; stirring rate = 200 - 800 rpm. The same conditions can be maintained with steroid transformations by genetically modified microorganisms in a multi-phase system. Suitable organic solvents are, among others, saturated an unsaturated alkanes, alcohols, esters and ethers of hydrocarbons and mixtures thereof since they have a high steroid solubility. Suitable microorganisms are particularly Pseudomonads, such as P. testosteroni, P. oleovorans and P. putida species, provided with the right genetic information.

## 8. Description of the drawings

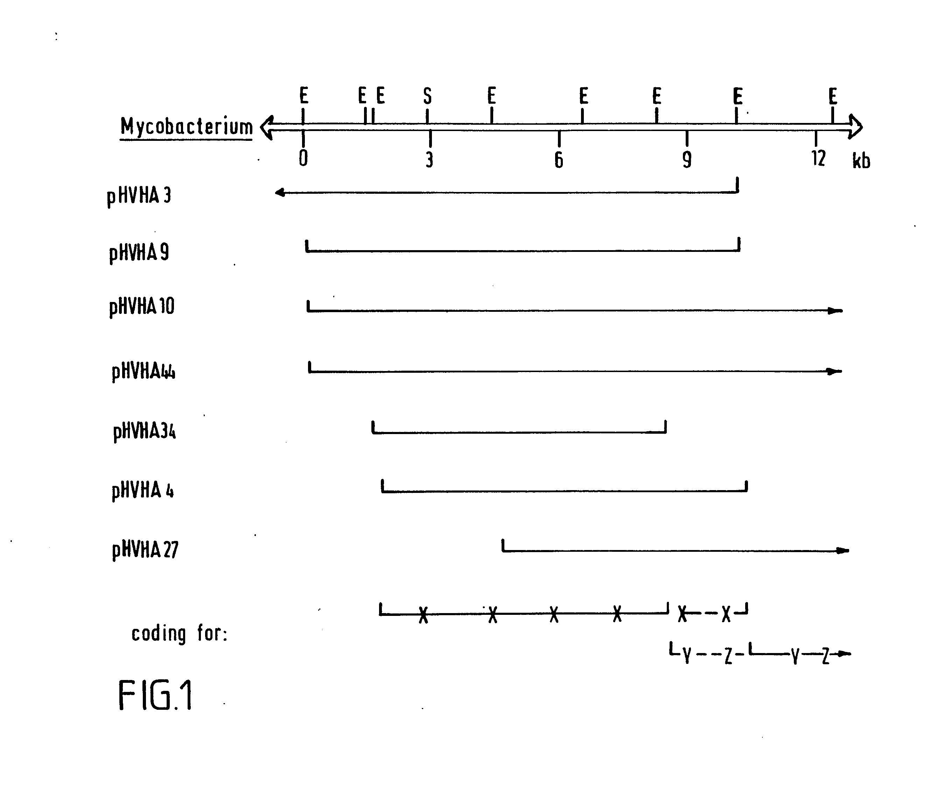

Fig. 1 shows the restriction map of a 12.45 kb Mycobacterium sp.NRRL-B 3683 DNA fragment coding for several steroid breakdown enzymes. For different recombinant plasmids it is indicated which part of the Mycobacterium DNA is inserted. The abbreviations used are: E = EcoRI restriction site; S = SaII restriction site; X = codes for growth on steroids; Y and Z = code for rapid nucleus degradation and product formation.

## 9. Deposit of strains

On December 19, 1988, the following strains were deposited with the Deutsche Sammlung von Mikroorganismen und Zellkulturen (DSM) of Braunschweig, FRG:
(a) an E. coli DH1 strain containing the plasmid pHVHA 10, and
(b) an E. coli DH1 strain containing the plasmid pHVHA 44.

## Claims

1. A process for preparing steroids, particularly C-17 ketosteroids, by means of steroid side chain oxidation through genetically modified microorganisms obtained by transfer of genetic material between two bacterial species.

2. A process according to claim 1, wherein the genetic information originates from a microorganism of the order Actinomycetales, particularly from Arthrobacter, Bacillus, Brevibacterium, Corynebacterium, Microbacterium, Mycobacterium, Nocardia, Protaminobacter, Rhodococcus, Serratia, or Streptomyces.

3. A process according to claim 1, wherein the host employed is a microorganism from the order Pseudomonads, particularly of the species Pseudomonas, more particularly Pseudomonas testosteroni, Pseudomonas oleovorans, or Pseudomonas putida.

4. A process according to claim 1, wherein the genetic information is inserted into the host by means of a plasmid vector, such as the cosmid pLAFRI which is stably inheritable in a wide range of hosts.

5. A process according to claim 1, wherein the genetic information inserted into the host comprises the plasmid pHVHA 10, the plasmid pHVHA 44, or a part thereof.

6. A process according to claim 1, wherein the substrate converted is a steroid having a C-17 side chain, more particularly a sterol, such as cholesterol, β-sitosterol, campesterol, stigmasterol, or a mixture comprising one or more of such sterols.

7. A process according to claim 1, wherein the conversion is carried out in a fermenter comprising an aqueous phase, an organic phase, or a multiphase system.

8. A process according to claim 1, wherein the conversion is carried out at a temperature of 18 - 42° C, preferably at 28 - 32° C, and at a pH value of 5 - 9, preferably 6.5 - 7.5.

9. A process according to claim 1, wherein the reactor is stirred at a rate of 200 - 1200 rpm, preferably a rate of 400 - 800 rpm.

10. A process according to claim 1, wherein the conversion is carried out in one or more organic solvents in which steroids dissolve better than in water, using a bacterial species, such as Pseudomonas oleovorans, or Pseudomonas putida, resistant to such organic solvents.

11. A process according to claim 1, wherein the conversion is carried out in one or more hydrocarbons, silicone compounds or perhalogenated hydrocarbons as solvents.

12. A process according to claim 1, wherein the conversion is carried out in an aqueous medium using a genetically engineered microorganism of a type other than the DNA donor, which microorganism can carry out the desired steroid conversion more rapidly or with less secondary reactions than the donor.

13. Genetically modified microorganisms obtained by transfer of genetic material between two bacterial species and capable of steroid side chain oxidation.

14. Genetically modified microorganisms according to claim 13, wherein the genetic information originates from a microorganism of the order Actinomycetales, particularly from Arthrobacter, Bacillus, Brevibacterium, Corynebacterium, Microbacterium, Mycobacterium, Nocardia, Protaminobacter, Rhododoccus, Serratia, or Streptomyces.

15. Genetically modified microorganisms according to claim 13, wherein the host employed is a microorganism from the order Pseudomonads, particularly of the species Pseudomonas, more particularly Pseudomonas testosteroni, Pseudomonas oleovorans, or Pseudomonas putida.

16. Genetically modified microorganisms according to claim 13, wherein the genetic information is inserted into the host by means of a plasmid vector, such as the cosmid pLAFRI, which is stably inheritable in a wide range of hosts.

17. Genetically modified microorganisms according to claim 13, wherein the genetic information inserted into the host originates from Mycobacterium sp. NRRL B-3683.

18. Genetically modified microorganisms according to claim 13, wherein the genetic information inserted into the host comprises the plasmid pHVHA 10, the plasmid pHVHA 44, or a part thereof.

FIG.1

EP 0 375 075 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 274 147 (NEDERLANDSE ORGANISATIE VOOR TOEGEPAST NATUURWETENSCHAPPELIJK ONDERZOEK) * Claims * --- | 1,2,6,7 | C 12 P 33/00 C 12 N 15/52 C 12 N 1/21 C 12 N 15/78 |
| A | CHEMICAL ABSTRACTS, vol. 108, no. 21, 25th May 1988, page 210, abstract no. 181468a, Columbus, Ohio, US; Y. MUROOKA et al.: "Novel plasmids and their use in manufacture of cholesterol oxidase with actinomycetes", & JP-A-62 285 789 (TOYOBO CO., LTD) 11-12-1987 --- | 1,2,13, 14 | C 12 P 1/00 // (C 12 N 1/21 C 12 R 1:38 ) |
| A | CHEMICAL ABSTRACTS, vol. 108, no. 17, 25th April 1988, page 391, abstract no. 146642f, Columbus, Ohio, US; H.J. STEINERT et al.: "Steroid side chain cleavage with immobilized living cells in organic solvents", & STUD. ORG. CHEM. (Amsterdam) 1987, 29(Biocatal. Org. Media), 51-63 --- | 7,10,11 | |
| A,D | THE JOURNAL OF BIOLOGICAL CHEMISTRY, vol. 262, no. 36, 25th December 1987, pages 17712-17718, Baltimore, MD, US; G. EGGINK et al.: "Controlled and functional expression of the Pseudomonas oleovorans alkane utilizing system in Pseudomonas putida and Escherichia coli" * Pages 17717-17718: discussion * --- | 4,10 | TECHNICAL FIELDS SEARCHED (Int. Cl.5) C 12 P C 12 N |
| A | EP-A-0 015 308 (HENKEL KGaA) * Claims * ----- | 1,2 | |

The present search report has been drawn up for all claims

| Place of search | Date of completion of the search | Examiner |
|---|---|---|
| THE HAGUE | 06-03-1990 | HUBER-MACK A. |